# DEMANDE DE BREVET EUROPEEN

(11) **EP 0 780 505 A2**
(43) Date de publication de la demande: **25.06.1997**
(21) Numéro de dépôt: 96490047.6
(22) Date de dépôt: 20.12.1996
(51) Int. Cl.: D04H 1/46, D04H 1/54

(54) **Partie femelle d'une fermeture auto-agrippante en non-tissé, son procédé de fabrication et fermeture agrippante ainsi obtenue**

(30) Priorité: 20.12.1995 FR 9515596
(71) Demandeur: Duflot Industrie (SA), 59157 Beauvois-en-Cambresis (FR)
(72) Inventeur: Kapuscik Jean, 59400 Fontaine Notre Dame (FR); Lemaire, Pierre, 59118 Wambrechies (FR)
(74) Mandataire: Hénnion, Jean-Claude

(57) **Abrégé**

Le matériau, destiné à former la partie femelle d'une fermeture auto-agrippante, est constitué d'un non-tissé aiguilleté et thermolié, composé d'un mélange de fibres de longueur moyenne d'au moins 50 mm, avec une proportion d'au moins 20 % de fibres thermoliantes, et ayant sur une face des boucles formées lors de l'aiguilletage.

Le procédé de fabrication consiste à :
a) réaliser une nappe de fibres (13) à partir d'un mélange comportant une proportion prédéterminée de fibres thermoliantes,
b) aiguilleter ladite nappe à l'aide d'aiguilles conventionnelles pour la consolidation de celle-ci et avec des aiguilles à fourche ou à couronne pour la formation de boucles (22) en surface, et
c) traiter thermiquement la nappe aiguilletée, sans calandrage, à une température suffisante pour obtenir la fusion superficielle des fibres thermoliantes.

## Description

La présente invention concerne le domaine des fermetures auto-agrippantes, qui sont bien connues notamment sous l'appellation Velcro, et qui comportent deux parties se présentant généralement sous forme de deux bandes, l'une portant des éléments agrippants, du type crochet, harpon ou tête de clou et l'autre qui porte des éléments aptes à être agrippés par les premiers et qui se présentent généralement sous la forme de boucles. La présente invention concerne plus particulièrement un matériau destiné à constituer la partie femelle d'une fermeture auto-agrippante, destinée notamment à être utilisée dans des produits consommables du type couches-culottes.

Dans le présent texte on utilise le terme "fermeture auto-agrippante" dans un sens large, comprenant le cas où les deux bandes équipées des éléments respectivement mâle et femelle sont intégrées dans les parties d'un même article qui ont à être solidarisées l'une à l'autre pour réaliser la fermeture dudit article, par exemple article d'habillement, dans la chaussure, et comprenant également le cas où il n'y a pas à proprement parler de fermeture d'un article donné mais simplement solidarisation de deux pièces distinctes dont chacune est équipée respectivement d'éléments mâle et femelle aptes à s'auto-agripper.

Les fermetures auto-agrippantes, notamment connues sous l'appellation Velcro, ont maintenant trouvé des applications très diverses dans les articles d'habillement, d'ameublement, dans la chaussure, dans les articles de loisirs... Elles présentent l'avantage d'une très grande simplicité de mise en oeuvre pour obtenir la fermeture par simple application des deux bandes l'une sur l'autre, une très grande résistance à l'arrachage sous traction dans le plan des deux bandes et une grande faculté de réutilisation sans détérioration excessive des éléments d'accrochage. Bien sûr la structure et la composition des éléments d'accrochage tiennent compte de l'utilisation qui est envisagée.

La bande femelle d'une fermeture auto-agrippante est généralement réalisée à partir d'un tricot, du type jersey, qui a subi une opération de finition du type brossage ou grattage destinée à libérer un certain nombre de fils en surface de manière à former des boucles.

Un tel matériau est notamment décrit dans la norme française NFG 91-101.

Le coût de fabrication d'un tel matériau, comprenant une opération de tricotage et une opération de finition, est élevé et n'est guère compatible avec l'utilisation de ce matériau dans un produit jetable et relativement bon marché que constitue une couche-culotte. On comprend également que dans un certain nombre d' utilisations, il n'est pas strictement nécessaire d'avoir une capacité très importante de réutilisation de la fermeture. Il est simplement nécessaire que celle-ci puisse être utilisée à une ou quelques reprises sans détérioration, mais avec une résistance suffisante à la traction dans le plan des deux bandes.

Le but que s'est fixé le demandeur est de proposer un matériau destiné à former la partie femelle d'une fermeture auto-agrippante qui répond à cet objectif.

Ce but est parfaitement atteint par le matériau de l'invention qui, de manière caractéristique, est constitué d'un non-tissé aiguilleté et thermolié, qui est composé d'un mélange de fibres de longueur moyenne d'au moins 50 mm, avec une portion d'au moins 20 % de fibres thermoliantes, ledit non-tissé ayant sur une face des boucles formées lors de l'aiguilletage.

Les boucles dépassant de la surface du non-tissé sont aptes à être agrippées par les éléments mâles de la fermeture. La longueur moyenne des fibres, faisant au moins 50 mm, et la présence des fibres thermoliantes permettent d'obtenir un ancrage suffisant des extrémités des boucles pour limiter le défibrage de celles-ci lors de l'utilisation.

La résistance mécanique de l'accrochage et donc l'efficacité du principe d'auto-agrippage est fonction du titrage des fibres mises en oeuvre dans le non-tissé. De préférence le titrage moyen est compris 1,7 et 17 dtex, fonction de la masse surfacique du non-tissé.

De préférence la longueur moyenne des fibres est comprise entre 60 et 100 mm.

Dans une variante préférée de réalisation, le non-tissé a une masse surfacique faisant de l'ordre de 100 g/m², le titrage moyen des fibres est de l'ordre de 4,5 dtex, la proportion des fibres du mélange étant de l'ordre de 30 % de fibres de copolyester et 70 % de fibres polyester.

Il peut être avantageux d'avoir, pour une masse surfacique donnée, une certaine proportion de fibres de faible titrage de manière à avoir une meilleure couverture du non-tissé.

C'est un autre objet de l'invention que de proposer un procédé spécialement conçu pour la fabrication d'un matériau à base de non-tissé aiguilleté thermolié destiné à former la partie femelle d'une fermeture auto-agrippante.

De manière caractéristique ce procédé consiste :
a) à réaliser une nappe de fibres à partir d'un mélange comportant une proportion prédéterminée de fibres thermoliantes,
b) à aiguilleter ladite nappe à l'aide d'aiguilles conventionnelles pour la consolidation de celle-ci et avec des aiguilles à fourche ou à couronne pour la formation de boucles en surface, et
c) à traiter thermiquement la nappe aiguilletée, de préférence sans calandrage, à une température suffisante pour obtenir la fusion superficielle des fibres thermoliantes.

Lors de la pénétration de l'aiguille à fourche à travers la nappe de fibres, une (ou plusieurs) fibre vient se loger dans la fente de l'aiguille et est entraînée par celle-ci. De même lors de la pénétration de l'aiguille à couronne, une (ou plusieurs) fibre vient se loger dans chacune des trois encoches réparties en couronne vers l'extrémité de l'aiguille, et est entraînée par celle-ci. La pénétration de l'aiguille est suffisante pour que la fibre en question soit repoussée au-delà de la surface extérieure de la nappe et forme une boucle, lors du retrait de l'aiguille. Le traitement thermique subséquent a pour effet de bloquer en position les boucles ainsi formées grâce aux points de liage réalisés par la fusion superficielle des fibres thermoliantes en contact entre elles ou avec d'autres fibres non thermoliantes. Cet effet de liage est obtenu par simple contact des fibres entre elles sans qu'il soit indispensable d'effectuer un calandrage. Il est à noter qu'un calandrage est à éviter, puisqu'il a pour effet d'écraser les boucles formées lors de l'aiguilletage et en conséquence de les rendre moins accessibles par les éléments agrippants de la partie mâle de la fermeture.

Cependant un calandrage peut être nécessaire lorsqu'il s'agit de réaliser la fusion superficielle des fibres thermoliantes et de solidariser le matériau de l'invention sur un film en une seule opération.

La formation des boucles lors de l'aiguilletage par les aiguilles à fourche ou à couronne dépend en grande partie de la disposition de ces aiguilles par rapport à la direction des fibres dans la nappe. Avantageusement, la nappe étant réalisée grâce à une carde alimentée par un nappeur, les aiguilles à fourche sont disposées de telle sorte que leur fente soit orientée sensiblement transversalement à la direction de déplacement de la nappe lors de l'aiguilletage. En effet le nappeur dispose les fibres avant l'entrée de la carde en couches transversales successives. Certes du fait du déplacement longitudinal de la nappe et du cardage, les fibres subissent une légère réorientation. Cependant l'efficacité de l'aiguilletage, en terme de boucles, est améliorée si les fentes des aiguilles ont l'orientation précitée par rapport à une orientation dans la direction du déplacement de la nappe.

Selon une variante préférée de réalisation, l'aiguilletage pour la consolidation de la nappe a été réalisé à l'aide d'aiguilles à barbes à raison de 105 coups/cm² et l'aiguilletage pour la formation des boucles a été réalisé à l'aide d'aiguilles à fourche à raison de l'ordre de 1 coup/cm².

Le non-tissé aiguilleté et thermolié présentant des boucles en surface, obtenu par le procédé précité, convient parfaitement pour une utilisation comme partie femelle d'une fermeture auto-agrippante. La densité des boucles, la profondeur d'aiguilletage, la proportion de fibres thermoliantes, le titrage des fibres, la longueur moyenne des fibres... sont des paramètres à prendre en considération en fonction notamment de la structure des éléments agrippants de la partie mâle de la fermeture. S'agissant en particulier d'une partie mâle dont les éléments agrippants ont une forme en tête de clou, la fermeture agrippante comporte également un non-tissé aiguilleté et thermolié selon l'invention dont les fibres ont un titrage moyen de 4,5 dtex et une longueur de fibres comprise entre 60 et 100 mm, la proportion de fibres thermoliantes étant de l'ordre de 30 %.

La présente invention sera mieux comprise à la lecture de la description qui va être faite d'une exemple de réalisation d'un non-tissé aiguilleté et thermolié à boucles apparentes destiné à constituer la partie femelle d'une fermeture auto-agrippante, illustré par le dessin annexé dans lequel :
- la figure 1 est une vue schématique en coupe de deux bandes constituant une fermeture auto-agrippante, à distance l'une de l'autre,
- la figure 2 est une vue schématique en coupe des deux bandes de la figure 1 appliquées partiellement l'une contre l'autre,
- la figure 3 est une vue de côté d'une aiguille à barbes,
- la figure 4 est une vue de côté d'une aiguille à fourche,
- la figure 5 est une représentation schématique illustrant la fabrication d'un non-tissé aiguilleté et thermolié à boucles apparentes,
- et la figure 6 est une représentation en perspective d'une couche-culotte munie d'un système de fermeture.

Une fermeture auto-agrippante 1 comporte de manière bien connue deux éléments, se présentant sous la forme de deux bandes ou rubans textiles 2,3 dont l'une 2 présente en surface des éléments 4. agrippants et dont l'autre 3 présente en surface des boucles 5. Ce type de fermeture auto-agrippante est maintenant tout-à-fait conventionnel, notamment connu sous l'appellation Velcro.

Les éléments agrippants 4 peuvent présenter diverses formes, du type crochet, harpon ou tête de clou.

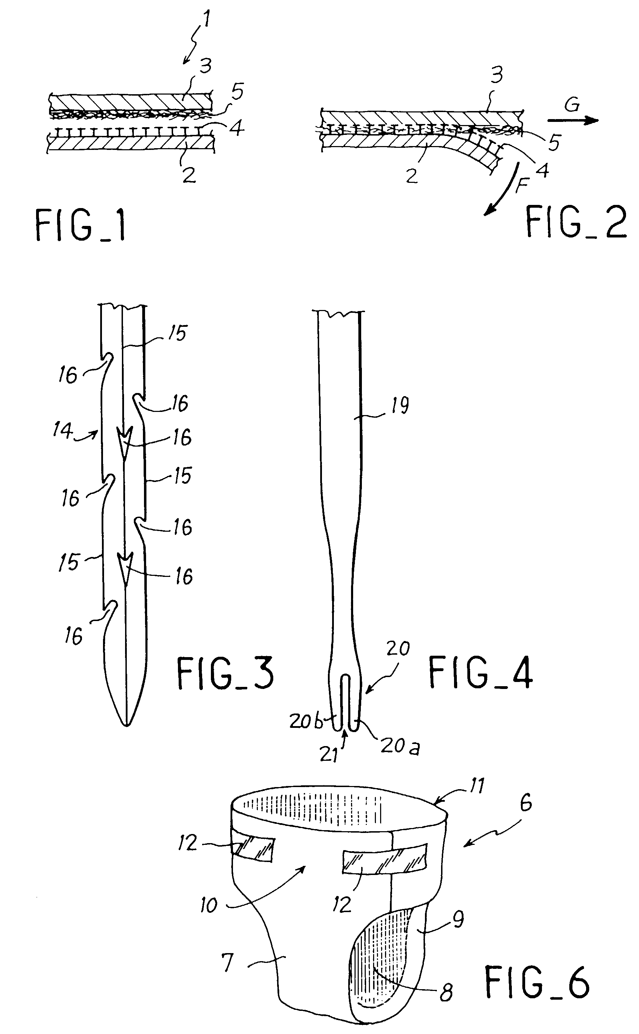

Lorsqu'on applique l'une contre l'autre les deux bandes 2, 3, les éléments agrippants 4 de la première bande 2 pénètrent dans les boucles 5 de la seconde bande 3. Du fait de la forme particulière des éléments agrippants 4 et de la multiplicité de ceux-ci on obtient un accrochage naturel des deux bandes 2, 3 grâce à cette pénétration. Il est bien sûr possible de désolidariser les deux bandes 2, 3 l'une de l'autre. Une telle désolidarisation est aisée lorsque l'on exerce une force en oblique par rapport au plan des deux bandes, comme illustré sur la figure 2 dans le sens de la flèche F. Par contre, cette force doit être notablement plus élevée si l'on veut désolidariser les deux bandes 2, 3 en exerçant une force dans le plan même des deux bandes, selon la flèche G.

On comprend que dans ces conditions une telle fermeture auto-agrippante est fréquemment utilisée dans toutes les applications où il s'agit de solidariser temporairement deux pièces entre lesquelles sont amenées à être exercées principalement des forces de traction dans le plan de solidarisation desdites pièces.

Ce type de fermeture auto-agrippante, connu principalement sous l'appellation Velcro, est utilisé dans le domaine de l'habillement, de l'ameublement, des loisirs, de la chaussure ...

On commence à proposer ce système de fermeture auto-agrippant également dans des produits jetables de grande consommation, tels que les couches-culottes. Une couche-culotte 6, telle que celle représentée à la figure 6, est constituée d'une pièce unique comportant une feuille extérieure imperméable 7, un matelas absorbant 8 et une feuille intérieure perméable 9, le matelas absorbant 9 étant pris en sandwich entre les deux feuilles extérieure 7 et intérieure 9. Après mise en place de la couche-culotte 6, les deux extrémités 10, 11 de celle-ci sont rabattues l'une vers l'autre autour de la taille de l'enfant. La fermeture de la couche-culotte est donc obtenue en solidarisant ces deux extrémités 10, 11 grâce généralement à un jeu de deux attaches adhésives 12.

Lors de la fabrication d'une couche-culotte, le jeu d'attache adhésive est préalablement fixé le long des bords latéraux d'une extrémité 11 de la couche 6, de sorte que pour la fermeture de la couche sur l'enfant il suffit à l'utilisateur de décoller une partie de l'attache adhésive, qui est appliquée sur une bande de protection, et de rapporter celle-ci sur la face extérieure de l'autre extrémité 10.

En matière d'attache adhésive, il existe une variété importante de solutions qui ont été apportées par les fabricants.

Dans le cadre de la présente invention, il est envisagé de remplacer le système d'attache adhésive pour la fermeture d'une telle couche-culotte 6, par un système de fermeture auto-agrippant.

Plus particulièrement l'objet de la présente invention est de proposer un matériau qui puisse convenir à constituer la partie femelle d'une fermeture auto-agrippante, c'est-à-dire la partie qui supporte des boucles apparentes sur une face.

Traditionnellement dans les fermetures auto-agrippantes connues, cette partie femelle est constituée par un tricot, du type jersey, dont le mode de tricotage permet, grâce à un traitement ultérieur de brossage et de grattage, de faire émerger des boucles en surface.

Pour une application dans un produit jetable tel qu'une couche-culotte, l'utilisation d'un tel support textile serait d'un coût prohibitif.

De manière caractéristique, selon l'invention, le matériau utilisé pour constituer la partie femelle de la fermeture auto-agrippante est un non-tissé aiguilleté et thermolié qui comporte des fibres ayant une longueur moyenne supérieure à 50 mm, une proportion de fibres thermoliantes d'au moins 20 % et qui présentent sur une face des boucles obtenues par aiguilletage.

Le demandeur a en effet remarqué qu'il était possible d'obtenir un ancrage des boucles dans la structure du non-tissé aiguilleté avec des fibres suffisamment longues, et en tout cas d'au moins 50 mm en moyenne, et avec un liage de ces fibres obtenu par un double effet d'aiguilletage et de thermoliage, la proportion des fibres thermoliantes étant d'au moins 20 %. Grâce à un tel ancrage, lorsqu'on désolidarise les deux parties mâle et femelle de la fermeture auto-agrippante, on limite le défibrage des boucles ce qui rend possible une réutilisation ultérieure de la partie femelle.

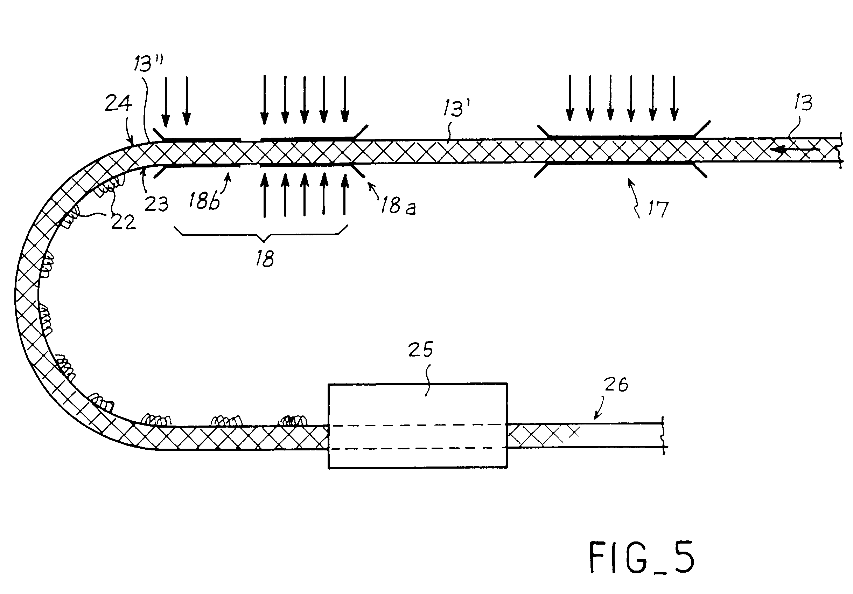

La figure 5 illustre le procédé de fabrication d'un tel non-tissé.

La nappe de fibres 13 est réalisée classiquement à l'aide d'un nappeur et d'une carde. Il s'agit par exemple d'une nappe constituée à partir de fibres de polyester à raison de 70 % et fibres de copolyester à raison de 30 %. Les fibres de copolyester sont des fibres thermoliantes qui ont un point de fusion inférieur à celui des fibres de polyester.

Dans un exemple précis de réalisation, la nappe 13 était réalisée à partir de 35 % de fibres polyester 3,3 dtex de 35 % de fibres polyester 6 dtex et 30 % de fibres copolyester 4,4 dtex. La longueur moyenne de ces trois types de fibres était de 60 mm. La nappe de fibres 13 passe d'abord dans un premier poste 17 d'aiguilletage à simple frappe, équipé d'aiguilles conventionnelles à barbes, telle qu'illustré à la figure 3. Ces aiguilles 14 comportent, selon chacune de leur arête 15, une succession d'encoches 16 réparties sur la périphérie de l'aiguille 14. Lors de la pénétration de l'aiguille 14 dans la nappe de fibres, les encoches 16 accrochent l'une ou l'autre fibre et la déplace à travers ladite nappe. Le déplacement est fonction de la pénétration de l'aiguille par rapport au support de la nappe.

Dans un exemple précis de réalisation, dans ce premier poste 17 d'aiguilletage, on avait une répartition de 5 666 aiguilles au mètre linéaire, les aiguilles étant du type 38 RB 3,5 pouces avec une densité d'aiguilletage de 55 coups/cm² pour une pénétration de 9 mm.

Cette première opération réalise une préconsolidation de la nappe.

La nappe 13' ainsi consolidée, passe ensuite dans un deuxième poste 18 d'aiguilletage à double frappe. Dans une première partie 18a dudit poste, il s'agit de poursuivre la consolidation de la nappe 13' avec un aiguilletage classique, double face, à l'aide d'aiguilles à barbes 14.

Dans un exemple précis de réalisation, cette première partie 18a du second poste d'aiguilletage 18 était munie sur la première face, correspondant à la frappe du premier poste 17, de 4 266 aiguilles par mètre linéaire, lesdites aiguilles étant des aiguilles conventionnelles à barbes 14 du type 36 R 3pouces. La densité d'aiguilletage était de 25 coups/cm² avec pénétration de 14 mm. Sur l'autre face, le poste 18 a été équipé d'également 4 266 aiguilles au mètre linéaire, s'agissant d'aiguilles du type 38 RB 3,5 pouces. La densité d'aiguilletage était de 25 coups/cm² avec une pénétration de 9 mm.

Tout-à-fait spécifiquement la seconde partie 18b du second poste d'aiguilletage 18 était équipé uniquement sur une face d'aiguilles à fourche 19, telles qu'illustrées à la figure 4. Il s'agit d'aiguilles dont l'extrémité libre 20 est séparée en deux parties parallèles 20a 20b, comme une fourche, délimitant entre elles une fente 21.

Lors de la pénétration de l'aiguille à travers la nappe, une ou plusieurs fibres sont emmenées par l'extrémité 20 de l'aiguille à fourche 19, la ou lesdites fibres venant se loger dans la fente 21. La pénétration de l'aiguille à fourche 19 est telle que la ou les fibres qui ont été entraînées par ladite aiguille se déplacent au-delà de la surface de la nappe 13' et forment des boucles 22 sur la face 23 opposée à la face 24 qui se trouve immédiatement en regard des aiguilles 19 dans la partie 18b du second poste 18 d'aiguilletage.

Selon le demandeur, la pénétration des aiguilles à fourche 19 ne devrait pas excéder 14 mm pour obtenir des boucles acceptables dans l'application envisagée.

De plus de manière à augmenter l'efficacité de captation par chaque aiguille à fourche 19 de fibres constitutives de la nappe 13, il est souhaitable que lesdites aiguilles 19 soient implantées dans la planche à aiguille de manière à ce que la fente 21 soit orientée sensiblement transversalement par rapport à la direction générale de déplacement de la nappe 13'. Cette disposition particulière vise à ce que la fente 21 ait la plus grande chance possible, à chaque coup, de se saisir d'une ou plusieurs fibres de la nappe. Ce résultat est obtenu dans les conditions précitées du fait que la direction générale des fibres dans la nappe est, après nappage et cardage, sensiblement transversale à la direction de déplacement de ladite nappe.

Dans un exemple précis de réalisation, les aiguilles à fourche 19 étaient des aiguilles du type 76 OVG. La densité d'aiguilletage était relativement faible, de l'ordre de ou légèrement inférieur à un coup/cm², avec une pénétration de l'ordre de 14 mm.

Dans les deux postes d'aiguilletage 17, 18, on avait réglé la vitesse d'aiguilletage à 1 054 coups/mn avec une vitesse d'avance de 18 m/mn pour la nappe de fibres et un pas d'aiguilletage de 17 mm.

Il est possible d'obtenir une surface 23 de nappe qui soit homogène quant à l'implantation des boucles 22. Il est également possible d'obtenir une implantation non homogène, avec un effet de bande transversale, simplement en équipant la partie 18b du second poste 18 d'aiguilletage avec un nombre d'aiguilles à fourche 19 inférieur au nombre maximal de la planche. Par exemple avec 4 rangées d'aiguilles on obtient un effet de dessins de bandes alternées, à intervalle régulier, sur la surface 23 de la nappe 13'.

Après passage dans ce second poste 18 d'aiguilletage, la nappe 13" est traitée thermiquement, sans calandrage ni écrasement, à une température suffisante pour obtenir la fusion superficielle des fibres thermoliantes.

Ce traitement thermique est par exemple obtenu par passage en continu de la nappe 13" à travers un four 25. Il peut s'agir d'un four à flux d'air chaud, par exemple un four à air pulsé de 8 m de longueur, l'air étant à une température de 205 °C et le temps de séjour de la nappe 13" dans le four étant de l'ordre d'une trentaine de seconde pour la vitesse de 18m/mn.

Bien qu'aucune pression ne soit exercée sur la nappe 13" alors que les fibres thermoliantes sont superficiellement fondues, on remarque que dans le non-tissé 26, sortant du four 25 il existe une multitude de points de liage au niveau des contacts entre les fibres thermoliantes 13 entre elles ou avec des fibres non thermoliantes, ces points de liage étant obtenus par simple contact des fibres entre elles.

Ces points de liage permettent, en complément de la consolidation par l'aiguilletage conventionnel, d'obtenir un ancrage des extrémités libres des fibres formant les boucles 22 à l'intérieur de la nappe 26 proprement dite.

On a mis en oeuvre le non-tissé 26 aiguilleté et thermolié, à boucle apparente, comme partie femelle 3 d'une fermeture auto-agrippante 1, avec comme élément agrippant 4 de la partie mâle 2 des éléments en forme de tête de clou.

Des essais de résistance à la rupture ont été réalisés, conformément à la norme NF G 91-106. Pour cela on a réalisé deux éprouvettes, l'une pour le non-tissé 26 de l'invention, formant un rectangle de 150 mm x 40 mm et l'autre de 120 mm de longueur pour un ruban mâle 2. On a appliqué une longueur de 30 mm de ruban mâle sur la face bouclée du non-tissé, chaque élément étant disposé entre les mâchoires d'un dynamomètre, distantes l'une de l'autre de 200 mm. Lesdites mâchoires ont été amenées à se déplacer à une vitesse de 100 mm/mn et on a mesuré la résistance moyenne et l'allongement moyen obtenus jusqu'à la désolidarisation du ruban mâle et du non-tissé. On a obtenu une résistance moyenne de 20 N et un allongement moyen de 7 %.

Dans l'application qui est envisagée du non-tissé 26 comme partie femelle d'une fermeture auto-agrippante pour couche-culotte, on conçoit que le non-tissé en question peut se présenter sous de multiple formes. Il peut consister simplement en une pièce rapportée sur la feuille extérieure imperméable 7 de la couche 6 en son extrémité 10, dimensionnée et localisée de manière à être précisément en vis-à-vis du ruban mâle constituant avec lui la fermeture auto-agrippante. Il peut également se présenter sous la forme d'une bande continue disposée transversalement sur toute la largeur de l'extrémité 10 de la couche 6, qui fera office de partie femelle pour les deux rubans mâles de fermeture de deux bords latéraux de la couche.

En fonction de la forme particulière de l'élément agrippant 4, le titrage moyen des fibres est à déterminer de manière à obtenir une efficacité suffisante du phénomène d'auto-agrippage. Selon le demandeur, ce titrage peut varier entre 1,7 dtex et 17 dtex en fonction de masse surfacique du non-tissé. Comme dans l'exemple précis de réalisation cité ci-dessus, le fait d'utiliser des fibres ayant un titrage plus faible peut améliorer la couverture du non-tissé.

Une augmentation de la proportion de fibres thermoliantes dans le mélange aura comme effet d'améliorer la résistance des fibres mais parallèlement d'augmenter la rigidité du non-tissé 26 obtenu.

La présente invention n'est pas limitée au mode de réalisation qui a été décrit à titre d'exemple non exhaustif. En particulier le non-tissé de l'invention peut être utilisé dans d'autres applications de produits jetables que la couche-culotte, pour lesquels il n'est pas nécessaire d'avoir la possibilité d'un ré-emploi fréquent.

Par exemple, dans le domaine des machines outils domestiques, telles que des ponceuses rotatives, le non-tissé de l'invention pourrait équiper la face lisse de chaque disque abrasif et coopérer avec les éléments mâles dont serait équipé le support rotatif de la ponceuse. On évite ainsi la mise en oeuvre de moyens mécaniques de blocage du disque abrasif sur le support.

De plus il est possible de mettre en oeuvre , en substitution des aiguilles à fourche , d'autres types d'aiguilles, en particulier des aiguilles dites à couronne, qui ont une structure générale à trois arêtes, similaire à celle de la figure 3, mais qui ne possèdent que trois encoches disposées au même niveau, vers l'extrémité de l'aiguille : leur action serait assimilable à celle de trois aiguilles à fourche accolées.

## Revendications

1. Matériau destiné à former la partie femelle d'une fermeture auto-agrippante caractérisé en ce qu'il est constitué d'un non-tissé aiguilleté et thermolié, qui est composé d'un mélange de fibres de longueur moyenne d'au moins 50 mm, avec une proportion d'au moins 20 % de fibres thermoliantes, ledit non-tissé ayant sur une face des boucles formées lors de l'aiguilletage.

2. Matériau selon la revendication 1 caractérisé en ce que le titrage moyen des fibres est compris entre 1,7 et 17 dtex.

3. Matériau selon l'une des revendications 1 ou 2 caractérisé en ce que la longueur moyenne des fibres est comprise entre 60 et 100 mm.

4. Matériau selon l'une des revendications 1 à 3 caractérisé en ce que le non-tissé a une masse surfacique faisant de l'ordre de 100 g/m², le titrage moyen des fibres est de l'ordre de 4,5 dtex, la proportion des fibres du mélange étant de l'ordre de 30 % de fibres de copolyester et 70 % de fibres polyester.

5. Procédé pour la fabrication d'un matériau à base de non-tissé aiguilleté thermolié destiné à former la partie femelle d'une fermeture auto-agrippante, caractérisé en ce qu'il consiste :
a) à réaliser une nappe de fibres (13) à partir d'un mélange comportant une proportion prédéterminée de fibres thermoliantes,
b) à aiguilleter ladite nappe à l'aide d'aiguilles conventionnelles (14) pour la consolidation de celle-ci et avec des aiguilles à fourche (19) ou à couronne pour la formation de boucles (22) en surface, et
c) à traiter thermiquement la nappe aiguilletée, à une température suffisante pour obtenir la fusion superficielle des fibres thermoliantes.

6. Procédé selon la revendication 5 caractérisé en ce que la nappe étant réalisée grâce à une carde alimentée par un nappeur, les aiguilles à fourche (19) sont disposées de telle sorte que leur fente (21) soit orientée sensiblement transversalement à la direction de déplacement de la nappe lors de l'aiguilletage.

7. Procédé selon l'une des revendications 5 ou 6 caractérisé en ce que l'aiguilletage pour la consolidation de la nappe a été réalisé à l'aide d'aiguilles à barbes à raison de 105 coups/cm² et l'aiguilletage pour la formation des boucles a été réalisé à l'aide d'aiguilles à fourche à raison de l'ordre de 1 coup/cm².

8. Procédé selon l'une des revendications 5 à 7 caractérisé en ce que le traitement thermique est réalisé par calandrage de la nappe aiguilletée et d'un film thermoplastique en sorte d'obtenir à la fois le thermoliage du non-tissé et la solidarisation du non-tissé du film.

9. Fermeture auto-agrippante composée d'une partie mâle dont les éléments agrippants ont une forme en tête de clou, et dont la partie femelle est un non-tissé aiguilleté et thermolié à boucles apparentes selon la revendication 1 dont les fibres ont un titrage moyen de 4,5 dtex et une longueur de fibres comprise entre 60 et 100 mm, la proportion de fibres thermoliantes étant de l'ordre de 30 %.
